# FASCICULE DE BREVET EUROPEEN

(11) **EP 1 754 455 B1**
(45) Date de publication et mention de la délivrance du brevet: **13.01.2010**
(21) Numéro de dépôt: 06291244.9
(22) Date de dépôt: 01.08.2006
(51) Int. Cl.: A61F 2/36

(54) **Partie fémorale d'une prothèse de hanche**
Femurteil einer Hüftprothese
Femoral part of a hip prosthesis

(30) Priorité: 02.08.2005 FR 0508225
(43) Date de publication de la demande: 21.02.2007
(73) Titulaire: Acor Tige Femorale, 49100 Angers (FR)
(72) Inventeur: Badatcheff, François, 49100 Angers (FR); Durand, Jean-Claude, 58000 Nevers (FR); Oufroukhi, Khamal, 49000 Saumur (FR); Atanasiu, Jean-Pierre, 86000 Poitiers (FR); Collet, Turialh, 44500 La Baule (FR); Decussac, Jean-Baptiste, 44500 La Baule (FR)
(74) Mandataire: Lavialle, Bruno François Stéphane

(56) Documents cités:
- FR-A- 2 606 273
- FR-A- 2 606 628
- FR-A- 2 647 669
- FR-A- 2 721 200

## Description

L'invention concerne une prothèse de hanche et plus particulièrement la partie fémorale de cette dernière.

### ARRIERE PLAN DE L'INVENTION

Une partie fémorale d'une prothèse de hanche selon les caractéristiques du préambule de la revendication 1 est connue du document FR-A-2 647 669.

La qualité d'un équipement prothétique pour la hanche notamment tient à la faculté de ce dernier à respecter le mieux possible l'anatomie du patient. Il s'agit donc de donner au chirurgien les moyens de réaliser le plus grand nombre possible de variations de la géométrie d'une prothèse, ce à partir d'éléments en nombre le plus réduit possible.

C'est ainsi qu'est apparue la modularité dans la prothèse de hanche et notamment dans la partie fémorale de cette dernière.

Généralement, la partie fémorale d'une prothèse de hanche modulaire comporte trois parties :
- une partie diaphysaire formant l'extrémité la plus éloignée de l'articulation,
- une partie métaphysaire qui se loge à l'endroit du fémur le plus évasé et
- un col implanté dans la partie métaphysaire pour supporter la sphère d'articulation.

Dans des cas plus rares, seul le col est modulaire, la tige (métaphyse et diaphyse) étant en une seule pièce. C'est notamment à cette réalisation que s'adresse l'invention.

Cet état de la technique est par exemple illustré par le document FR 2 721 200-A1 dans lequel la partie de col de la prothèse est rapportée dans une cavité dite "sensiblement elliptique" de la partie supérieure de la tige.

En réalité cette partie est formée par un massif dont la surface extérieure est segmentée en deux parties sensiblement coniques qui forment des extrémités du massif du col logé dans la tige, et deux parties planes convergentes l'une vers l'autre qui forment les parties latérales de ce massif. Les pentes mises en oeuvre dans ces surfaces sont conformes à celles connues en mécanique avec des emmanchements dits à cône morse. En outre, ce massif d'emmanchement est prolongé par un axe qui coopère avec un orifice cylindrique borgne ménagé au fond de la cavité de la partie de tige.

Cette géométrie présente plusieurs inconvénients. L'un d'eux réside dans l'existence d'un double centrage entre le massif et la cavité d'une part et l'axe et l'orifice cylindrique borgne d'autre part, qu'il est en toute rigueur impossible de réaliser correctement compte tenu des imprécisions et des incertitudes dimensionnelles de fabrication des deux pièces devant être emmanchées. Ces imprécisions requièrent alors de procéder à des apairements entre col et tige, qui sont très coûteux au plan de la fabrication et qui interdisent une réversibilité de l'emmanchement sauf au détriment de la qualité de ce dernier (micro-mouvement possible entre la tige et le col de la prothèse).

Un second inconvénient tient au profil conjugué du massif et le la cavité. La section radiale de ces massif et cavité est oblongue avec deux côtés parallèles rectilignes et deux segments circulaires les réunissant en extrémité. Il existe des arêtes à la jonction de ces quatre éléments périphériques de la section car les segments circulaires d'extrémité seraient de rayon trop petit s'ils étaient tangents aux côtés latéraux de la section. Il se trouve que dans les petites tailles de prothèse, la dimension transversale du massif ou de la prothèse peut atteindre des valeurs qui ne soient plus compatibles avec les charges à supporter.

### OBJET DE L'INVENTION

La présente invention entend remédier à ces inconvénients en proposant une prothèse de hanche dont la partie fémorale est à col modulaire implantable dans l'extrémité supérieure de la partie de tige.

### BREVE DESCRIPTION DE L'INVENTION

A cet effet donc, la partie fémorale d'une prothèse de hanche selon l'invention comporte une tige dont la partie supérieure est pourvue d'une cavité pour recevoir par emmanchement l'extrémité d'un col en forme de massif de forme correspondant à celle de la cavité, l'autre extrémité du col comportant un cône mâle de réception pour une sphère d'articulation, la cavité et le massif étant, vus en section perpendiculaire à la direction d'emmanchement, limités par quatre segments de cercle diamétralement opposés et raccordés en tangence deux à deux, définissant ainsi une forme oblongue dont les extrémités arrondies ont un rayon six à sept fois plus petit que celui des segments latéraux.

L'expérience a montré que la forme de cette section correspondait bien à un minimum de matière pour un maximum de résistance, notamment de résistance à la fermeture de l'angle que forme la tige de la prothèse avec le col (autour de 140°), en offrant à la liaison col-tige une grande surface de contact au travers de laquelle, du côté concave de la prothèse, les efforts passant du col à la tige, nés du poids du patient prothésé, engendrent des contraintes plus faibles que dans les prothèses connues. Il s'ensuit que l'on peut, toujours du côté concave de la prothèse, amincir la paroi de la cavité, sans que cette paroi perde en résistance.

L'expérimentation a montré que les dimensions optimales de la section susdite du massif et de la cavité, prises à l'entrée de la cavité, sont de 17 et 9 millimètres selon respectivement le grand axe et le petit axe de cette section, tandis que les petits rayons sont de 4 millimètres alors que les grands rayons sont de 24,5 millimètres.

En outre, les parois latérales de la cavité du massif ont une pente sur la direction d'emmanchement d'un angle sensiblement égal à 2°50'. On réalise ainsi un emmanchement du type cône-morse extrêmement résistant et insensible aux micro-mouvements des deux pièces l'une par rapport à l'autre.

De manière préférée le massif du col est encastrable dans la cavité selon deux orientations angulairement opposées autour de la direction d'emmanchement. Ces deux positions permettent donc d'obtenir avec un seul col deux tiges de prothèse différentes si l'axe du cône de support de la sphère d'articulation n'est pas confondu avec l'axe d'emmanchement.

De manière préférée dans le cas des dimensions susdites, la hauteur d'encastrement du cône dans la cavité est de l'ordre de 15 mm.

Enfin le cône de réception de la sphère est un tronc de cône dont la petite base a un diamètre de 10 mm et la grande base un diamètre de 12 mm, l'angle au sommet de ce cône étant égal à 5°42'30".

D'autres caractéristiques et avantages de l'invention ressortiront de la description donnée ci-après à titre d'exemple non limitatif.

### BREVE DESCRIPTION DES DESSINS

Il sera fait référence aux dessins annexés parmi lesquels :
- la figure 1 est une vue éclatée partielle d'une tige fémorale de prothèse de hanche conforme à l'invention,
- la figure 2 est une représentation de la section d'encastrement d'un col et d'une partie de tige de la prothèse, ladite section étant prise dans un plan perpendiculaire à la direction d'emmanchement,
- la figure 3 illustre par une coupe un col implanté dans la partie de tige de la prothèse.

### DESCRIPTION DETAILLEE DE L'INVENTION

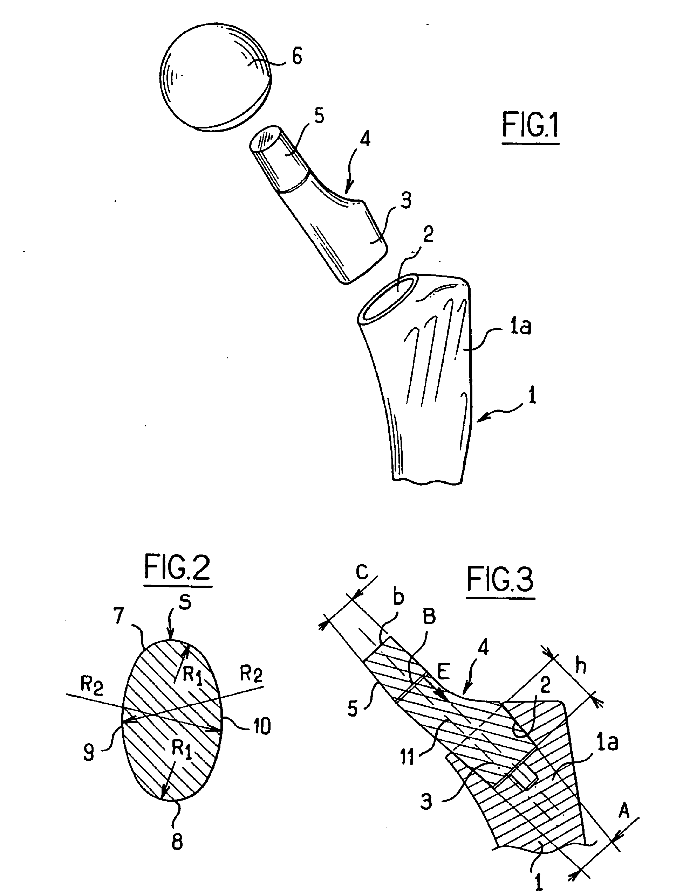

A la figure 1 on a représenté uniquement la partie métaphysaire 1a de la tige 1 de prothèse selon l'invention. Cette partie métaphysaire 1a possède une cavité 2 dans laquelle est susceptible d'être emmanché le massif 3 d'un col modulaire 4. A l'opposé du massif 3, le col modulaire comporte un cône 5 qui peut être coiffé par une sphère d'articulation 6.

La section courante de la cavité 2 ou du massif 3 du col 4 qui s'y emmanche, est représentée à la figure 2 par le contour S. Il s'agit d'une section oblongue limitée par quatre segments de cercle opposés deux à deux, dont deux segments 7 et 8 de petit rayon R₁ et deux segments 9 et 10 de grand rayon R₂. Ces segments sont raccordés les uns aux autres par tangence de sorte que la section ne présente aucune partie anguleuse. De manière préférée selon l'invention, le rayon R₁ des segments 7 et 8 est environ six à sept fois plus petit que le rayon R₂ des segments 9 et 10. A titre d'exemple, on indiquera que, au débouché de la cavité 2, le rayon R₁ vaut 4 mm tandis que le rayon R₂ vaut 24,5 mm. En outre, la section du massif et de la cavité diminue dans la direction d'emmanchement E représentée à la figure 3. La diminution est telle que la paroi latérale du massif 3 ou la paroi latérale de la cavité 2 converge vers cette direction E d'un angle égal à 2°50'. En d'autres termes, la surface conique d'emmanchement ayant pour grande base la section correspondant au contour S a un angle au sommet A de valeur égale à 5°40'. La hauteur h de la cavité est de l'ordre de 15 mm de manière à permettre un emmanchement sensiblement égal à cette valeur à quelques dixièmes de millimètre près.

On rappellera que le cône 5 qui est coiffé par la sphère 6 possède une petite base b de diamètre égal à 10 mm et une grande base B de diamètre égal à 12 mm. Son angle au sommet C est quant à lui égal à 5°42'30".

La surface extérieure du col comprise entre le cône 5 et le massif 3 est de finition telle qu'elle présente un poli-miroir, ce qui permet de limiter l'usure par cette surface de la cupule en polyéthylène qui vient coiffer la sphère 6.

A la figure 3, on a représenté le col 4 d'une prothèse latéralisée. Le plan de coupe étant le plan principal de la tige et du col, l'axe 11 du cône 5 est décalé par rapport à la direction d'emmanchement E d'une valeur définissant la latéralisation du col. Une fois la prothèse assemblée, contrairement aux prothèses antéversées ou rétroversées, l'axe 11 appartient au plan principal de la tige, c'est-à-dire au plan de coupe de la figure 3.

## Revendications

1. Partie fémorale d'une prothèse de hanche comportant une tige (1) dont la partie supérieure (la) est pourvue d'une cavité (2) pour recevoir par emmanchement le massif (3) d'extrémité de forme correspondante d'un col (4) dont l'autre extrémité comporte un cône mâle (5) de réception pour une sphère (6) d'articulation, la cavité (2) et le massif (3) sont, pour leur section (S) perpendiculaire à la direction (E) d'emmanchement, limités par quatre segments (7, 8, 9, 10) de cercle diamétralement opposés et raccordés en tangence deux à deux, définissant ainsi une forme oblongue, **caractérisée en ce que** les extrémités arrondies (7, 8) de ladite forme oblongue ont un rayon (R1) six à sept fois plus petit que celui (R2) des segments latéraux (9, 10).

2. Partie fémorale selon la revendication 1, **caractérisée en ce que** les dimensions de la section (S) susdite, prises à l'entrée de la cavité (2), sont de 17 et 9 mm selon respectivement son grand axe et son petit axe, tandis que le petit rayon est de 4 mm alors que le grand rayon est de 24,5 mm.

3. Partie fémorale selon l'une quelconque des revendications précédentes, **caractérisée en ce que** les parois latérales de la cavité et du massif ont une pente sur la direction d'emmanchement d'un angle sensiblement égal à 2°50'.

4. Partie fémorale selon l'une quelconque des revendications précédentes, **caractérisée en ce que** le massif (3) du col (4) est encastrable dans la cavité (2) selon deux orientations angulairement opposées autour de la direction (E) d'emmanchement.

5. Partie fémorale selon l'une quelconque des revendications 2 à 4, **caractérisée en ce que** la hauteur (h) de la cavité est de l'ordre de 15 mm.

6. Partie fémoral selon l'une quelconque des revendications précédentes, **caractérisée en ce que** le cône (5) de réception de la sphère (6) est un tronc de cône dont le diamètre de la petite base (b) est égal à 10 mm alors que le diamètre de la grande base (B) est égal à 12 mm, l'angle (C) au sommet de ce tronc de cône étant égal à 5°42'30".

## Claims

1. A femoral portion of a hip prosthesis, comprising a shaft (1) having an upper portion (1a) that is provided with a cavity (2) for receiving, by push fitting, the solid end body (3) with a corresponding shape of a neck (4)the other end of which comprises a male cone (5) for receiving a ball joint (6), the cavity (2) and the solid body (3), in their section (S) perpendicular to the direction (E) of push fitting, being defined by four segments (7, 8, 9, 10) of a circle that are diametrically opposite and connected tangentially in pairs, thereby defining an oblong shape, **characterized in that** the rounded ends (7, 8) of said oblong shape have a radius (R₁) that is six to seven times smaller than the radius (R₂) of the lateral segments (9, 10).

2. A femoral portion according to claim 1, **characterized in that** the dimensions of said section (S) at the inlet to the cavity (2) are 17 mm and 9 mm along its major axis and minor axis respectively, while the small radius is 4 mm and the large radius is 24.5 mm.

3. A femoral portion according to any preceding claim, **characterized in that** the lateral walls of the cavity and the solid body are inclined to the direction of push fitting by an angle substantially equal to 2°50'.

4. A femoral portion according to any preceding claim, **characterized in that** the solid body (3) of the neck (4) can be installed in the cavity (2) in two angularly opposite orientations about the direction (E) of push fitting.

5. A femoral portion according to any one of claims 2 to 4, **characterized in that** the height (h) of the cavity is of the order of 15 mm.

6. A femoral portion according to any preceding claim, **characterized in that** the cone (5) for receiving the ball (6) is a truncated cone with a minor base (b) of diameter equal to 10 mm while the major base (B) has a diameter equal to 12 mm, the cone angle (C) of said truncated cone being equal to 5°42'30".

## Patentansprüche

1. Femurteil einer Hüftprothese, umfassend eine Stange (1), deren oberer Abschnitt (1a) mit einem Hohlraum (2) versehen ist, um durch Eindrücken den Endstumpf (3) entsprechender Form eines Halses (4) aufzunehmen, dessen anderes Ende einen männlichen Kegel (5) zur Aufnahme einer Gelenkkugel (6) umfasst, wobei der Hohlraum (2) und der Stumpf (3) bezüglich ihres Querschnittes (S), der senkrecht zur Eindrückrichtung (E) ist, durch vier diametral gegenüberliegende und paarweise tangential verbundene Kreisabschnitte (7, 8, 9, 10) begrenzt sind, die somit eine längliche Form festlegen, **dadurch gekennzeichnet, dass** die abgerundeten Enden (7, 8) der genannten länglichen Form einen Radius (R1) haben, der sechs bis sieben mal kleiner als der Radius (R2) der seitlichen Segmente (9, 10) ist.

2. Femurteil nach Anspruch 1, **dadurch gekennzeichnet, dass** die Abmessungen des oben genannten Querschnitts (S) gemessen am Eingang des Hohlraums (2) 17 und 9 mm betragen, entlang seiner großen bzw. seiner kleinen Achse, wohingegen der kleine Radius 4 mm beträgt, während der große Radius 24,5 mm beträgt.

3. Femurteil nach einem der vorhergehenden Ansprüche, **dadurch gekennzeichnet, dass** die seitlichen Wände des Hohlraums und des Stumpfs eine Neigung zur Eindrückrichtung mit einem Winkel von im Wesentlichen gleich 2°50' haben.

4. Femurteil nach einem der vorhergehenden Ansprüche, **dadurch gekennzeichnet, dass** der Stumpf (3) des Halses (4) in dem Hohlraum (2) in zwei Ausrichtungen eingesetzt werden kann, die um die Eindrückrichtung (E) herum in Winkelrichtung entgegengesetzt sind.

5. Femurteil nach einem der Ansprüche 2 bis 4, **dadurch gekennzeichnet, dass** die Höhe (h) des Hohlraums in der Größenordnung von 15 mm liegt.

6. Femurteil nach einem der vorhergehenden Ansprüche, **dadurch gekennzeichnet, dass** der Kegel (5) zur Aufnahme der Kugel (6) ein Kegelstumpf ist, dessen Durchmesser der kleinen Basis (b) gleich 10 mm ist, während der Durchmesser der großen Basis (B) gleich 12 mm ist, wobei der Winkel (C) an der Spitze dieses Kegelstumpfes gleich 5°42'30" ist.
